(19) Europäisches Patentamt
European Patent Office
Office européen des brevets

(11) Veröffentlichungsnummer: **0 138 773**
**B1**

(12) **EUROPÄISCHE PATENTSCHRIFT**

(45) Veröffentlichungstag der Patentschrift: **29.08.90**

(21) Anmeldenummer: **84810502.9**

(22) Anmeldetag: **12.10.84**

(51) Int. Cl.$^5$: **C 07 D 215/26,**
C 07 D 215/28,
C 07 D 405/12, A 01 N 43/42

(54) **Verwendung von Chinolinderivaten zum Schützen von Kulturpflanzen.**

(30) Priorität: **18.10.83 CH 5647/83**

(43) Veröffentlichungstag der Anmeldung:
**24.04.85 Patentblatt 85/17**

(45) Bekanntmachung des Hinweises auf die Patenterteilung:
**29.08.90 Patentblatt 90/35**

(84) Benannte Vertragsstaaten:
**AT BE CH DE FR GB IT LI NL**

(56) Entgegenhaltungen:
**EP-A-0 086 750**
**EP-A-0 094 349**
**DE-A-2 546 845**
**FR-A-1 538 541**
**FR-A-2 257 580**
**US-A-3 351 525**

**C.A. 101, 211479e**
**C.A. 91, 192861e**
**J.Het. Chem. 4(1967), S. 131**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Martin, Henry, Dr.**
**Schützenmattstrasse 52**
**CH-4051 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 138 773 B1**

Courier Press, Leamington Spa, England.

**Beschreibung**

Die vorliegende Erfindung betrifft die Verwendung von Chinolinderivaten zum Schützen von Kulturpflanzen gegen schädigende Wirkungen von Herbiziden, Mittel, welche diese Chinolinderivate enthalten, neue Chinolinderivate und die Herstellung der neuen Chinolinderivate.

Beim Einsatz von Herbiziden, können in Abhängigkeit von Faktoren wie beispeilsweise Dosis des Herbizids und Applikationsart, Art der Kulturpflanze, Bodenbeschaffenheit und klimatischen Bedingungen, wie beispielsweise Belichtungsdauer, Temperatur und Niederschlagsmengen, die Kulturpflanzen in gewissem Masse geschädigt werden. So ist beispielsweise bekannt, dass Herbizide aus den verschiedensten Stoffklassen, wie Triazine, Harnstoffderivate, Carbamate, Thiolcarbamate, Halogenacetanilide. Halogenphenoxyessigsäuren und anderen Klassen bei der Anwendung in wirksamer Dosis die Kulturpflanzen, welche gegen die nachteilige Workung von unerwünschtem Pflanzenwuchs geschützt werden sollen, in gewissem Masse schädigen können. Um diesem Problem zu begegnen, sind schon verschiedene Stoffe vorgeschlagen worden, welche befähigt sind, die schädigende Wirkung eines Herbizids aud die Kulturpflanze spezifisch zu antagonisieren, d.h., die Kulturpflanze zu schützen, ohne zugleich die Herbizidwirkung gegen zu bekämpfende Unkräuter merklich zu beeinflussen. Dabie hat sich jedoch gezeigt, dass die vorgeschlagenen Gegenmittel häufig nur eine enges Einsatzgebiet haben, d.h., ein bestimmtes Gegenmittel eignet sich oftmals nur zur Anwendung bei einzelnen Kulturpflanzenarten und/oder zum Schutz der Kulturpflanzen gegen einzelne herbizide Substanzen oder Stoffklassen.

So beschriebt die GB—A—1,277,557 die Behandlung von Samen bzw. Sprösslingen von Weizen und Sorghum mit gewissen Oxamsäureestern und Amiden zum Schutz vor dem Angriff durch "ALACHLOR" (N-Methoxymethyl-N-chloracetyl-2,6-diäthylanilin). In den DE—A—1,952,910 und DE—A—2,245,471, sowie in der FR—A—2,021,611 werden Gegenmittel zur Behandlung von Getreide-, Mais- und Reissamen zum Schutz gegen die schädigende Einwirkung von herbizid wirksamen Thiolcarbamaten vorgeschlagen. Gemäss der DE—C—1,567,075 und der US—A—3,131,509 werden Hydroxyaminoacetanilide und Hydantoine für den Schutz von Getreidesamen gegenüber Carbamaten verwendet.

Die direkte pre- oder postemergente Behandlung gewisser Nutzpflanzen mit Gegenmitteln als Antagonisten bestimmter Herbizidklassen auf einer Anbaufläche ist in den DE—A—2,141,586 und DE—A—2,218,097, sowie im US—A—3,867,444 beschrieben.

Ferner können Maispflanzen gemäss der DE—A—2,402,983 wirksam vor Schädigung durch Chloracetanilide geschützt werden, indem man dem Boden als Gegenmittel ein N-disubstituiertes Dichloracetamid zuführt.

Gemäss EP—A—11,047 können auch Alkoximinobenzylcyanide, deren Alkoxygruppe u.a. durch eine acetalisierte Carbonylgruppe substituiert ist, als Mittel zum Schutz von Kulturpflanzen vor der schädigenden Wirkung von Herbiziden verschiedener Stoffklassen verwendet werden.

Weiterhin beschrieben beispielsweise die US—A—4,176,185, die britischen Patentschriften 760,319, 989,578, 1,003,477 und 1,003,478, die CH—A—408,007, die DE—A—25 46 845, Areschka, A. et al., Eur. J. Med. Chem.-Chimica Therapeutica, Sept.-Okt. 1975—*10*, No. 5, 463—469, Major R. T. et al., J. Med. Pharm. Chem. 4, 317—326, 1961, und Thompson, H. E. Botan. Gaz. 107. 476—507, 1946, den Einsatz von Chinolinderivaten auf therapeutischem Gebiet, als Ausgangsprodukt zur Herstellung therapeutischer Wirkstoffe, als Mittel zur Wachstumsförderung bei Tieren, als Pflanzenwuchshemmer oder als Herbizide.

Die Verwendung bestimmter Chinolin-8-yl-oxy-alkylcarbonsäure-Nitrile und -Amidoxime als Safener gegen die kulturpflanzenschädigende Wirkung von Herbiziden ist aus der EP—A—0 086 750 bekannt geworden.

Weiterhin betrifft die nicht vorveröffentlichte EP—A—0 094 349 die Verwendung von Chinolin-8-yl-oxy-alkylcarbonsäuren und deren Derivate als Herbizid Antidote.

Aus der FR—B—1,538,541 sind verschiedene 8-Benzyloxychinoline bekannt geworden. Es wird deren Verwendung als Pflanzenfungizide vorgeschlagen.

In Godeng Xaexiao Huaxue Xuebao *5* (1984), 526—8 (Chem. Abstr. *101*: 211479e) wird die Verwendung von 8-(3,7-Dimethyl-oct-6-enyloxy)-chinolin als Insekten-Juvenilhormon analoge Verbindung beschrieben.

Ebenfalls als Inksekten-Juvenilhormon ist 8-(3,7-Dimethylocta-2,6-dienyloxy)-chinolin aus Indian J. Chem. Sect. B, *17B* (1979) 54—6 (Chem. Abstr. *91*: 192861 e) bekannt geworden.

8-Allyloxychinolin wird in mehreren wissenschaftlichen Publikationen beschrieben. Unter anderem auch in der FR—B—2257580 als Zwischenverbindung bei der Herstellung von photographischen Entwicklern.

Untersuchungen zur Allyloxyumlagerung von 8-Allyloxy-4-methylchinolin und 8-Allyloxy-2-methylchinolin sind aus J. Het. Chem. *4* (1967) 131—2 bekannt.

Es wurde nun gefunden, dass sich überraschenderweise eine Gruppe von Chinolinderivaten hervorragend dazu eignet, Kulturpflanzen gegten schädigende Wirkungen von Herbiziden, zu schützen. Diese Chinolinderivate werden daher im folgenden auch als "Gegenmittel", "Antidot" oder "Safener" bezeichnet.

Chinolinderivate, welche zum Schützen von Kulturpflanzen gegen schädigende Wirkungen von Herbiziden geeignet sind, entsprechen der Formel I

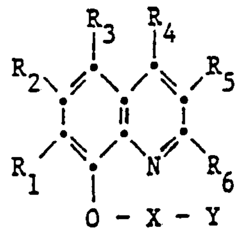

(I),

worin

$R_1$, $R_2$ und $R_3$ unabhangig voneinander Wasserstoff, Halogen, Nitro, Cyan, $C_1$—$C_4$-Alkyl, $C_1$—$C_4$-Alkoxy oder $C_1$—$C_3$-Acyl,

$R_4$, $R_5$ und $R_6$ unabhängig voneinander Wasserstoff, Halogen oder $C_1$—$C_4$-Alkyl,

X und Y zusammen einen aliphatischen, gesättigten oder ungesättigten geradkettigen oder verzweigten Kohlenwasserstoffrest mit bis zu 12 Kohlenstoffatom, mit der Massgabe, dass im Falle der ungesättigten Reste die Mehrfachbindung durch mindestens eine an der Mehrfachbindung nicht beteiligtes Kohlenstoffatom vom Sauerstoffatom getrennt ist, oder

X Methylen und

Y einen Dioxolanrest, einen Dioxanrest oder einen Tetrahydrofuranrest, die gegebenenfalls durch Alkyl mit 1 bis 4 C-Atomen substituiert sind, bedeutet, unter Einschluss ihrer Säureadditionssalze und Metallkomplexe.

Soweit optisch insomere Verbindungen der Formel I existieren, sind im Rahmen der vorliegenden Erfindung sowohl die optisch reinen Isomere wie auch die Isomerengemische zu verstehen.

In den Verbindungen der Formel I sind unter Halgen Fluor, Chlor, Brom und Jod zu verstehen, insbesondere Chlor, Brom und Jod.

Die als Substituenten $R_1$, $R_2$, $R_3$, $R_4$, $R_5$ und $R_6$ vorkommenden Alkyl- und Alkoxygruppen können geradkettig oder verzweigt sein enthalten 1 bis 4, und insbesondere 1 bis 3 Kohlenstoffatome. Beispiele für solche Substituenten sind Methyl, Aethyl, n-Propyl, Isopropyl, sowie Methoxy, Aethoxy, Isopropoxy, n-Propoxy oder Butoxy.

Als $C_1$—$C_3$-Acylreste kommen Alkylcarbonylgruppen mit 1—3 C-Atomen in Betracht. Diese Acylreste bilden auch bevorzugt den Acylteil der Acyloxygruppe. Hier sind beispielsweise die Propionyloxy- und die Acetoxygruppe zu nennen.

Wenn die Substituentengruppe —X—Y zusammen gesättigte oder ungesättigte, geradkettige oder verzweigte Kohlenwasserstoffreste mit zu 12 C-Atomen bedeutet kommenen beispielsweise in Frage: Methyl, Aethyl, n-Propyl, Isopropyl, n-Butyl, Isobutyl, sek.-Butyl, tert.-Butyl, Allyl, Butenyl, Methallyl, Dimethallyl, Chlorallyl, Propargyl, n-Pentyl, Isopentyl, n-Hexyl, n-Heptyl, n-Oktyl und n-Dodecyl und Isomere davon.

Bei den über Sauerstoff gebundenen, umgesättigten Kohlenwasserstoffresten ist die Mehrfachbindung durch mindestens ein an der Mehrfachbindung nicht beteiligtes Kohlenstoffatom vom Sauerstoffatom getrennt.

Beispiel für Tetrahydrofuranyl, Dioxanyl und Dioxolanyl sind. Tetrahydrofuran-2-yl, 1,3-Dioxan-2-yl, 1,3-Dioxolan-2-yl, gegebenenfalls durch Alkyl mit 1 bis 4 Kohlenstoffatomen substituiertes Dioxolanyl ist beispielsweise 2-Methyl-1,3-dioxolan-4-yl.

Als Salzbildner kommen organische und anorganische Säuren in Betracht. Beispiele organischer Säuren sind Essigsäure, Trichloressigsäure, Oxalsäure, Benzolsulfonsäure und Methansulfonsäure. Beispiele anorganischer Säuren sind Chlorwasserstoffsäure, Bromwasserstoffsäure, Jodwasserstoffsäure, Schwefelsäure, Phosphorsäure, phosphorige Säure und Salpetersäure.

Als Metallkomplexbildner eignen sich beispielsweise Elemente der 3. und 4. Hauptgruppe, wie Aluminum, Zinn und Blei, sowie der 1. bis 8. Nebengruppe, wie beispielsweise Chrom, Mangan, Eisen, Kobalt, Nickel, Zirkon, Zink, Kupfer, Silber und Quecksilber. Bevorzugt sind die Nebengruppenelemente der 4. Periode.

Besonders geeignet zur erfindungsgemässen Verwendung sind Verbindungen der Formel I, die den nachfolgend aufgeführten Verbindungsgruppen angehören:

a) Verbindungen der Formel I, in denen $R_1$, $R_2$, $R_3$, $R_4$, $R_5$ und $R_6$ die für Formel I angegebenen Bedeutungen haben und X und Y zusammen einen aliphatischen, gesättigten oder ungesättigten Kohlenwasserstoffrest bilden, unter Einschluss ihrer Säureadditionssalze und Metallkomplexe;

b) Verbindungen der Formel I, in denen $R_1$, $R_2$, $R_3$, $R_4$, $R_5$ und $R_6$ die für Formel I angegebenen Bedeutungen haben und X und Y zusammen einen aliphatischen, gesättigten Kohlenwasserstoffrest bilden, unter Einschluss ihrer Säureadditionssalze und Metallkomplexe;

c) Verbindungen der Formel I, in denen $R_1$, $R_2$, $R_3$, $R_4$, $R_5$ und $R_6$ die für Formel I angegebenen Bedeutungen haben und X und Y zusammen einen aliphatischen, ungesättigten Kohlenwasserstoffrest bilden, unter Einschluss ihrer Säureadditionssalze und Metallkomplexe;

d) Verbindungen der Formel I, in denen $R_3$ Halogen bedeutet, $R_1$, $R_2$, $R_4$, $R_5$ und $R_6$ die für Formel I

angegebenen Bedeutungen haben und X und Y zusammen den n-Octylrest bilden, unter Einschluss ihrer Säureadditionssalze und Metallkomplexe;

e) Verbindungen der Formel I, in denen $R_3$ Chlor bedeutet, $R_1$, $R_2$, $R_4$, $R_5$ und $R_6$ die für Formel I angegebenen Bedeutungen haben und X und Y zusammen den n-Octylrest bilden, unter Einschluss der Säureadditionssalze und Metallkomplexe;

f) Verbindungen der Formel I, in denen $R_3$ Halogen bedeutet, $R_1$, $R_2$, $R_4$, $R_5$ und $R_6$ die für Formel I angegebenen Bedeutungen haben und X und Y zusammen den 2-Butenyl-Rest bilden, unter Einschluss der Säureadditionssalze und Metallkomplexe;

g) Verbindungen der Formel I, in denen $R_3$ Chlor bedeutet, $R_1$, $R_2$, $R_4$, $R_5$ und $R_6$ die für Formel I angegebenen Bedeutungen haben und X und Y zusammen den 2-Butenyl-Rest bilden, unter Einschluss der Säureadditionssalze und Metallkomplexe;

h) Verbindungen der Formel I, in denen $R_3$ Chlor, und $R_1$, $R_2$, $R_4$, $R_5$ und $R_6$ Wasserstoff bedeuten und X und Y zusammen den n-Octylrest bilden, unter Einschluss der Säureadditionssalze und Metallkomplexe;

i) Verbindungen der Formel I, in denen $R_3$ Chlor und $R_1$, $R_2$, $R_4$, $R_5$ und $R_6$ Wasserstoff bedeuten X und Y zusammen den 2-Butenyl-Rest bilden, unter Einschluss der Säureadditionssalze und Metallkomplexe;

k) Verbindungen der Formel I, in denen $R_1$, $R_2$, $R_3$, $R_4$, $R_5$ und $R_6$ die für Formel I angegebene Bedeutung haben und X Methylen und Y einen Dioxolanrest, einen Dioxanrest oder eine Tetrahydrofuranrest bedeuten, unter Einschluss ihrer Säureadditionssalze und Metallkomplexe;

l) Verbindungen der Formel I, in denen $R_3$ Halogen und $R_1$, $R_2$, $R_4$, $R_5$ und $R_6$ Wasserstoff bedeuten und X Methylen und Y einen Dioxolanrest, einen Dioxanrest oder einen Tetrahydrofuranrest bedeuten, unter Einschluss ihrer Säureadditionssalze und Metallkomplexe;

m) Verbindungen der Formel I, in denen $R_3$ Chlor und $R_1$, $R_2$, $R_4$, $R_5$ und $R_6$ Wasserstoff bedeuten und X Methylen und Y einen Dioxolanrest, einen Dioxanrest oder einen Tetrahydrofuranrest bedeuten, unter Einschluss ihrer Säureadditionssalze und Metallkomplexe;

n) Verbindungen der Formel I, in denen $R_3$ Chlor und $R_1$, $R_2$, $R_4$, $R_5$ und $R_6$ Wasserstoff bedeuten und X Methylen und Y einen Dioxolanrest bedeuten, unter Einschluss der Säureadditionssalze und Metallkomplexe;

o) Verbindungen der Formel I, in denen $R_3$ Chlor und $R_1$, $R_2$, $R_4$, $R_5$ und $R_6$ Wasserstoff und X Methylen und Y einen Dioxanrest bedeutet, unter Einschluss der Säureadditionssalze und Metallkomplexe;

p) Verbindungen der Formel I, in denen $R_3$ Chlor und $R_1$, $R_2$, $R_4$, $R_5$ und $R_6$ Wasserstoff und X Methylen und Y einen Tetrahydrofuranrest bedeutet, unter Einschluss der Säureadditionssalze und Metallkomplexe;

q) Verbindungen der Formel I, in denen $R_1$, $R_2$, $R_3$, $R_4$, $R_5$ und $R_6$ Wasserstoff bedeuten und X und Y zusammen einen aliphatischen gesättigten oder ungesättigten Kohlenwasserstoffrest bilden, unter Einschluss ihrer Säureadditionssalze und Metallkomplexe;

r) Verbindungen der Formel I, in denen $R_1$, $R_2$, $R_3$, $R_4$, $R_5$ und $R_6$ Wasserstoff bedeuten und X und Y zusammen den 2-Butenyl-Rest bilden, unter Einschluss der Säureadditionssalze und Metallkomplexe;

s) Verbindungen der Formel I, in denen $R_1$, $R_2$, $R_3$, $R_4$, $R_5$ und $R_6$ Wasserstoff, X Methylen und Y einen Dioxolanrest, einen Dioxanrest oder einen Tetrahydrofuranrest bedeuten, unter Einschluss ihrer Säureadditionssalze und Metallkomplexe;

t) Verbindungen der Formel I, in denen $R_1$, $R_2$, $R_3$, $R_4$, $R_5$ und $R_6$ Wasserstoff, X Methylen und Y einen Dioxolrest bedeuten, unter Einschluss ihrer Säureadditionssalze und Metallkomplexe;

u) Verbindungen de Formel I, in denen $R_1$, $R_2$, $R_3$, $R_4$, $R_5$ und $R_6$ Wasserstoff, X Methylen und Y einen Dioxanrest bedeuten, untere Einschluss ihrer Säureadditionssalze und Metallkomplexe.

v) Verbindungen der Formel I, in denen $R_1$, $R_2$, $R_3$, $R_4$, $R_5$ und $R_6$ Wasserstoff, X Methylen und Y einen Tetrahydrofuranrest bedeuten, unter Einschluss ihrer Säureadditionssalze und Metallkomplexe;

w) Verbindungen r Formel I, in denen $R_1$, $R_2$, $R_4$ und $R_5$ Wasserstoff, $R_3$ Wasserstoff oder Chlor und $R_6$ Wasserstoff oder Methyl bedeuten und die Substituentengruppe —X—Y für n-Oktyl, Allyl, 2-Butenyl, Propargyl, 1,3-Dioxolan-2-ylmethyl, 1,3-Dioxan-2-ylmethyl oder Tetrahydrofuran-2-ylmethyl steht.

x) Verbindungen, in denen $R_1$ und $R_3$ unabhängig voneinander Wasserstoff oder Chlor, $R_2$, $R_4$ und $R_5$ Wasserstoff, $R_6$ Wasserstoff oder Methyl, X Methylen und Y 1,3-Dioxolan-2-yl, 1,3-Dioxolan-4-yl, 2-Methyl-1,3-dioxolan-4-yl, oder 1,3-Dioxan-2-yl, bedeuten.

y) Verbindungen, in denen $R_1$, $R_2$, $R_4$, $R_5$ und $R_6$ Wasserstoff, $R_3$ Chlor und Y Wasserstoff und X und Y gemeinsam n-Butyl, sek-Butyl, 2-Propenyl, 2-Butenyl oder 2-Propinyl bedeuten.

z) Verbindungen der Formel I worin $R_1$—$R_6$ die für Formel I angebene Bedeutung haben und die Substituentengruppe —X—Y 2-Butenyl, 1,3-Dioxolan-2-ylmethyl, 1,3-Dioxan-2-ylmethyl oder Tetrahydrofuran-2-ylmethyl bedeutet.

1,3-Dioxolan-2-ylmethyl, 1,3-Dioxan-2-ylmethyl oder Tetrahydrofuran-2-ylmethyl bedeutet.

Wegen ihrer Wirkung bevorzugte Einzelverbindungen:

8-Allyloxychinolin,
8-(2-Butenyloxy)-chinolin,
5-Chlor-8-allyloxychinolin,
5-Chlor-8-propargyloxychinolin,
5-Chlor-8-(2-butenyloxy)-chinaldin,
8-Oktyloxychinolin,
5-Chlor-8-oktyloxychinolin,

8-Oktyloxychinaldin,
5-Chlor-8-oktyloxychinaldin,
8-(1,3-Dioxolan-2-ylmethoxy)-chinolin,
5-Chlor-8-(1,3-dioxolan-2-ylmethoxy)-chinolin,
8-(1,3-Dioxan-2-ylmethoxy)-chinolin,
5-Chlor-8-(1,3-dioxan-2-ylmethoxy)-chinolin,
5-Chlor-8-(tetrahydrofuran-2-ylmethoxy)-chinolin und insbesondere
5-Chlor-8-(2-butenyloxy)-chinolin.

Die Chinolinderivate der Formel I besitzen in hervorragendem Masse die Eigenschaft, Kulturpflanzen gegen schädigende Wirkungen von Herbiziden zu schützen. Die Herbizide können beispielsweise zu einer der folgenden Stoffklassen gehören: Triazine und Triazinone; Phenylharnstoffe, insbesondere 3-(4-Isopropylphenyl)-1,1-dimethylharnstoff ("Isoproturon"); Carbamate und Thiocarbamate; Halogenacetanilide, insbesondere Chloracetanilide; Chloracetamide; Halogenphenoxyessigsäureester; Diphenyläther, wie beispielsweise substituierte Phenoxyphenoxyessigsäureester und -amide und substituierte Phenoxyphenoxypropionsäureester und -amide; substituierte Pyridyloxyphenoxyessigsäureester und -amide und substituierte Pyridyloxyphenoxypropionsäureester und -amide, insbesondere 2-[4-(3,5-Dichlorpyridyl-2-oxy)-phenoxy]-propionsäure-2-propinylester; Benzoesäurederivate; Nitroaniline; Oxadiazolone; Sulfonylharnstoff; (4,5-Dihydro-4-oxo-1H-imidazol-2-yl)-benzosäure-Derivate, -nicotinsäure-Derivate und -chinolincarbonsäure-Derivate; Phosphate; und Pyrazole.

Im einzelnen kommen beispeilsweise folgende Substanzen in Betracht:

*Triazine und Triazinone*: 2,4-Bis(isopropylamino)-6-methylthio-1,3,5-triazin ("Prometryn"), 2,4-bis(äthylamino)-6-methylthio-1,3,5-triazin ("Simetryn"), 2-(1',2'-Dimethylpropylamino)-4-äthylamino-6-methylthio-1,3,5-triazin ("Dimethametryn"), 4-Amino-6-tert.butyl-4,5-dihydro-3-methylthio-1,2,4-triazin-5-on ("Metribuzin").

*Phenylharnstoff*: N-(3',4'-Dimethylbenzyl)-N'-4-tolyl-harnstoff (RDimuron), N-(3'-Chlor-4'-isopropylphenyl)-N',N'-(3-methyl-pentamethylen-1,5-yl)-harnstoff.

*Carbamate und Thiocarbamate*: N-(3',4'-Dichlorphenyl)-propionanilid ("propanil"), S-4-Chlorbenzyl-diäthyl-thiocarbamat ("Benthiocarb"), S-Aethyl-N,N-hexamethylen-thiocarbamat ("Molinate"), S-Aethyldi-propyl-thiocarbamat ("EPTC"), N,N-di-sec. Butyl-S-benzyl-thiocarbamat (RDrepamon), S-(2,3-Dichlorallyl)-di-isopropyl-thiocarbamat ("Di-allate"), 1-(Propylthiocarbonyl)-decahydro-chinaldin.

*Chloracetanilide*: 2-Chlor-2',6'-diäthyl-N-(2''-propyloxyäthyl)acetanilid ("Propalochlor"), 2-Chlor-6'-äthyl-N-(2''-methoxy-1''-methyläthyl)-acet-o-toluidid ("Metolachlor"), 2-Chlor-2',6'-diäthyl-N-(butoxymethyl)acetanilid ("Butachlor"), 2-Chlor-6'-äthyl-N-(äthoxymethyl)acet-o-toluidid ("Acetochlor"), 2-Chlor-6'-äthyl-N-(2''-propoxy-1''-methyläthyl)acet-o-toluidid, 2-Chlor-2,6'-dimethyl-N-(2''-methoxy-1''-methyläthyl)acetanilid, 2-Chlor-2',6'-dimethyl-N-(2''-methoxyäthyl)acetanilid ("Dimethachlor"), 2-Chlor-2',6'-diäthyl-N-(pyrazol-1-ylmethyl)acetanilid, 2-Chlor-6'-äthyl-N-(pyrazol-1-ylmethyl)acet-o-toluidid, 2-Chlor-6'-äthyl-N-(3,5-dimethyl-pyrazol-1-ylmethyl)acet-o-toluidid, 2-Chlor-6'-äthyl-N-(2''-butoxy-1''-methyläthyl)-acet-o-toluidid ("Metazolachlor"), 2-Chlor-6'-äthyl-N-(2''-butoxy-1''-(methyläthyl)acet-o-toluidid und 2-Chlor-2'-trimethylsilyl-N-(butoxymethyl)-acetanilid.

*Chloracetamide*: N-[1-Isopropyl-2-methylpropen-1-yl-(1)]-N-(2'-methoxyäthyl)-chloracetamid.

*Diphenyläther und Nitrodiphenyläther*: 2,4-Dichlorphenyl-4'-nitrophenyläther ("Nitrofen"), 2-Chlor-1-(3'-äthoxy-4'-nitrophenoxy)-4-trifluormethyl-benzol ("Oxyfluorfen"), 2',4'-Dichlorphenyl-3-methoxy-4-nitrophenyl-äther ("Chlormethoxynil"), 2-[4'-(2'',4''-Dichlorphenoxy)phenoxy)propionsäure-methylester, N-(2'-Phenoxyäthyl)-2-[5'(2''-chlor-4''-trifluoromethylphenoxy)-phenoxy]-propionsäureamid.

*Benzoesäurederivate*: Methyl-5-(2',4'-dichlorphenoxy)-2-nitrobenzoat ("Bifenox"), 5-(2'-Chlor-4'-trifluormethylphenoxy)-2-nitrobenzoesäure ("Acifluorfen"), 2,6-Dichlorbenzonitril ("Dichlobenil").

*Nitroaniline*: 2,6-Dinitro-N,N-dipropyl-4-trifluormethylanilin ("Trifluralin"), N-(1'-Aethylpropyl)-2,6-dinitro-3,4-xyliden ("Pendimethalin").

*Oxadiazolone*: 5-tert.-Butyl-3-(2',4'-dichlor-5'-isopropoxyphenyl)-1,3,4-oxadiazol-2-on ("Oxadiazon").

*Phosphate*: S-2-Methylpiperidino-carbonylmethyl-O,O-dipropyl-phosphorodithioat ("Piperophos").

*Pyrazole*: 1,3-Dimethyl-4-(2',4'-dichlorbenzoyl)-5-(4'-tolylsulfonyloxy)-pyrazol.

Besonders geeignet sind die Verbindungen der Formel I zum Schützen von Kulturpflanzen gegen schädigende Wirkungen von Herbiziden der Formel H—I

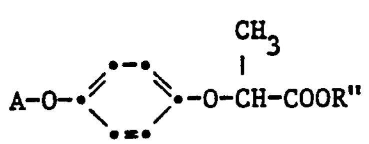

(H-I)

worin A 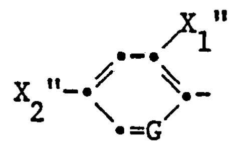 , 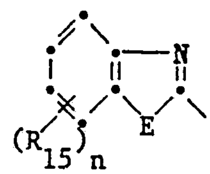 oder 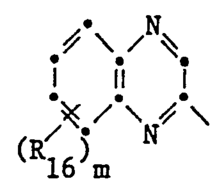 ,

R'' $C_1$—$C_4$-Alkyl, welches unsubstituiert oder durch $C_1$—$C_4$-Alkoxy substituiert ist, $C_3$—$C_4$-Alkenyl, $C_3$—$C_4$-Alkinyl oder

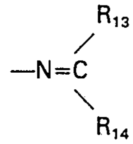 ,

$R_{13}$ $C_1$—$C_4$-Alkyl, $R_{14}$ $C_1$—$C_4$-Alkyl oder $R_{13}$ und $R_{14}$ gemeinsam $C_4$—$C_5$-Alkylen,
$X_1$'' Wasserstoff oder Halogen,
$X_2$'' Wasserstoff, Halogen oder Trifluormethyl,
G das Fragment =N— oder =CH—,
E ein Sauerstoff- oder Schwefelatom,
$R_{15}$ Halogen, $C_1$—$C_4$-Alkyl oder Trifluormethyl,
n eine Zahl von 0 bis 2,
$R_{16}$ Halogen, $C_1$—$C_4$-Alkyl oder Trifluormethyl und
m eine Zahl von 0 bis 2 bedeuten.

Zu betonen ist auch die gute Schutzwirkung von Verbindungen der Formel I bei Kulturpflanzen, insbesondere bei Getreide, gegen schädigende Wirkungen von Herbiziden, wie Diphenyläther-Derivaten und substituierten Pyridyloxyphenoxypropionsäureestern, insbesondere 2-[4-(3,5-Dichlorpyridyl-2-oxy)-phenoxy]-propionsäure-2-propinylester.

Als Kulturpflanzen, welche durch Chinolinderivate der Formel I gegen Herbizide geschützt werden können, kommen insbesondere diejenigen in Betracht, die auf dem Nahrungs- oder Textilsektor von Bedeutung sind, wie beispielsweise Kulturhirse, Reis, Mais, Getreidearten (Weizen, Roggen, Gerste, Hafer), Baumwolle, Zuckerrüben, Zuckerrohr und Soja.

Ein geeignetes Verfahren zum Schützen von Kulturpflanzen unter Verwendung von Verbindungen der Formel I besteht darin, dass man Kulturpflanzen, Teile, dieser Pflanzen oder für den Anbau der Kulturpflanzen bestimmte Böden vor oder nach dem Einbringen des pflanzlichen Materials in den Boden mit einer Verbindung der Formel I oder einem Mittel, welches eine solche Verbindung enthält, behandelt. Die Behandlung kann vor, gleichzeitig mit oder nach dem Einsatz des Herbizids erfolgen. Als Pflanzenteile kommen insbesondere diejenigen in Betracht, die zur Neubildung einer Pflanze befähigt sind, wie beispielsweise Samen, Früchte, Stengelteile und Zweige (Stecklinge) sowie auch Wurzeln, Knollen und Rhizome.

Die Erfindung betrifft auch ein Verfahren zur selektiven Bekämpfung von Unkräutern in Kulturpflanzenbeständen, wobei die Kulturpflanzenbestände, Teile der Kulturpflanzen oder Anbauflächen für Kulturpflanzen mit einem Herbizid und einer Verbindung der Formel I oder einem Mittel, welches diese Kombination enthält, behandelt werden. Die die Herbzid/Antidot-Kombination enthaltenden Mittel bilden ebenfalls einen Bestandteil der vorliegenden Erfindung.

Bei dun zu bekämpfenden Unkräutern kann es sich sowohl um monokotyle wie um dikotyle Umkräuter handeln.

Als Kulturpflanzen oder Teile dieser Pflanzen kommen beispielsweise die vorstehend genannten in Betracht. Als Anbauflächen gelten die bereits mit Kulturpflanzen bewachsenen oder die ausgesäten Bodenareale, wie auch die zur Bebauung mit Kulturpflanzen bestimmten Böden.

Die zu applizierende Aufwandmenge Antidot im Verhältnis zum Herbizid richtet sich weitgehend nach der Anwendungsart. Bei einer Feldehandlung, welche entweder unter Verwendung einer Tankmischung oder durch getrennte Applikation von Herbizid und Antidot durchgeführt wird, liegt in der Regel ein Verhältnis von Antidot zu Herbizid von 1:100 bis 10:1, bevorzugt 1:5 bis 8:1, und insbesondere 1:1, vor.

Dagegen werden bei der Samenbeizung und ähnlichen Einsatzmethoden weit geringere Mengen Antidot im Verhältnis zur Aufwandmenge an Herbizid/ha Anbaufläche benötigt. Bei der Samenbeizung werden in der Regel 0,1 bis 10 g Antidot/kg Samen, bevorzugt 1 bis 2 g, appliziert. Wird das Gegenmittel kurz vor der Aussaat unter Samenquellung appliziert, so werden zweckmässigerweise Antidot-Lösungen verwendet, welche den Wirkstoff in einer Konzentration von 1 bis 10 000 ppm, bevorzugt 100 bis 1000 ppm, enthalten.

Die Verbindungen der Formel I können für sich allein oder zusammen mit inerten Zusatzstoffen und/oder den zu antagonisierenden Herbiziden zur Anwendung gelangen.

Die vorliegende Anmeldung betrifft daher auch Mittel, welche Verbindungen der Formel I und inerte Zusatzstoff und/oder zu antagonisierende Herbizide, enthalten.

Zur Applikation werden die Verbindungen der Formel I oder Kombinationen von Verbindungen der Formel I mit zu antagonisierenden Herbiziden zweckmässigerweise zusammen mit den in der Formulierungstechnik üblichen Hilfsmitteln eingesetzt und werden daher z.B. zu Emulsionskonzentration, streichfähigen Pasten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt.

Die Formulierungen, d.h., die den Wirkstoff der Formel I oder eine Kombination von Wirkstoff der Formel I mit zu antagonisierendem Herbizid und gegebenenfalls einen festen oder flüssigen Zusatzstoff enthaltenden Mittel, Zubereitungen oder zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffs der Formel I und gegebenenfalls auch des zu antagonisierenden Herbizids nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$—$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyltaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxyde-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Pripylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsaltze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood New Jersey, 1980 Stache, H., "Tensid-Taschenbuch", Carl Hanser Verlag, München/Wien, 1981

Die agrochemischen zubereitungen enthalten in der Regel 0,1 bis 99 Gewichtsprozent, insbesondere 0,1 bis 95 Gewichtsprozent, Wirkstoff der Formel I oder eine Kombination von Wirkstoff der Formel I mit zu antagonisierendem Herbizid, 1 bis 99,9 Gewichtsprozent, insbesondere 5 bis 99,8 Gewichtsprozent, eines festen oder flüssigen Zusatzstoffes und 0 bis 25 Gewichtsprozent, insbesondere 0,1 bis 25 Gewichtsprozent, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Für die Verwendung von Verbindungen der Formel I oder sie enthaltender Mittel zum Schützen von Kulturpflanzen gegen schädigende Wirkungen von Herbiziden kommen verschiedene Methoden und Techniken in Betracht, wie beispielsweise die folgenden:

i) Samenbeizung

a) Beizung der Samen mit einem als Spritzpulver formulierten Wirkstoff durch Schütteln in einem Gefäss bis zur gleichmässigen Verteilung auf der Samenoberfläche (Trockenbeizung). Man verwendet dabei etwa 10 bis 500 g Wirkstoff der Formel I (40 g bis 2 kg Spritzpulver) pro 100 kg Saatgut.

b) Beizung der Samen mit einem Emulsionskonzentrat des Wirkstoffs der Formel I nach der Methode a) (Nassbeizung).

c) Beizung durch Tauchen des Saatguts in eine Brühe mit 50—3200 ppm Wirkstoff der Formel I während 1 bis 72 Stunden und gegebenenfalls nachfolgendes Trocknen der Samen (Tauchbeizung).

Die Beizung des Saatguts oder die Behandlung des angekeimten Sämlings sind naturgemäss die bevorzugten Metoden der Applikation, weil die Wirkstoffbehandlung vollständig auf die Zielkultur gerichtet ist. Man verwendet in der Regel 10 g bis 500 g, vorzugsweise 50 bis 250 g AS pro 100 kg Saatgut, wobei man je nach Methodik, die auch den Zusatz anderer Wirkstoff oder Mikronährstoffe ermöglicht, von den angegebenen Grenzkonzentrationen nach oben oder unter abweichen kann (Wiederholungsbeize).

ii) Applikation aus Tankmischung

Eine flüssige Aufarbeitung eines Gemisches von Gegenmittel und Herbizid (gegenseitiges Mengenverhältnis zwischen 10:1 und 1:10) wird verwendet, wobei die Aufwandmenge an Herbizid 0,1 bis 10 kg pro Hektar beträgt. Solche Tankmischung wird vorzugsweise vor oder unmittelbar nach der Aussaat appliziert oder 5 bis 10 cm tief in den noch nicht gesäten Boden eingearbeitet.

iii) Applikation in die Saatfurche

Das Gegenmittel wird als Emulsionskonzentrat, Spritzpulver oder als Granulat in die offene besäte Saatfurche eingebracht und hierauf wird nach dem Decken der Saatfurche in normaler Weise das Herbizid im Vorauflaufverfahren appliziert.

iv) Kontrollierte Wirkstoffabgabe

Der Wirkstoff wird in Lösung auf mineralische Granulatträger oder polymerisierte Granulate (Harnstoff/Formaldehyd) aufgezogen und trocknen gelassen. Gegebenenfalls kann ein Ueberzug aufgebracht werden (Umhüllungsgranulate), der es erlaubt, den Wirkstoff über einen bestimmten Zeitraum dosiert abzugeben.

Für einen Teil der unter der Formel I fallenden Chinolinderivate sind Verfahren zu ihrer Herstellung bekannt, sowie auch die Verwendung als Bakterizide und Fungizide. Die Verwendung der Chinolinderivate der Formel I als Safener ist jedoch nicht bekannt und erschliesst somit ein neuartiges Einsatzgebiet.

Einen Bestandteil der vorliegenden Anmeldung bilden neue Chinolinderivate der Formel I.

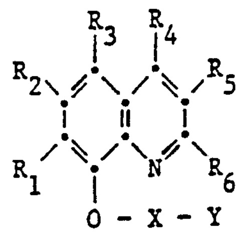

(I),

worin die Reste $R_1$ bis $R_6$, X und Y wie in Formel I definiert sind unter Einschluss ihrer Säureadditionssalze und Metallkomplexe, mit der Massgabe, dass nachstehend genannte Verbindungen nicht umfasst sind;

8-(3,7-Dimethyl-oct-6-enyl-oxy)-chinolin,
8-(3,7-Dimethyl-octa-2,6-dienyl-oxy)-chinolin,
8-Allyloxychinolin, 8-Allyloxy-4-methylchinolin und 8-Allyloxy-2-methylchinolin.

Besonders erwähnenswert sind neue Verbindungen der Formel Ia

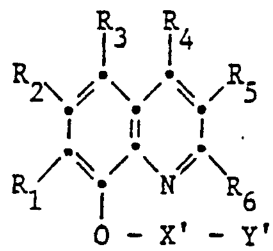

(Ia),

worin die Reste $R_1$ bis $R_6$ wie zuvor definiert sind und die Substituentengruppe —X'—Y' 2-Butenyl, 1,3-Dioxolan-2-ylmethyl, 1,3-Dioxan-2-ylmethyl oder Tetrahydrofuran-2-ylmethyl bedeuten.

Von den neuen Verbindungen der Formel I sind besonders diejenigen erwähnenswert, welche den Gegenstand einer des als bevorzugt angegebenen Verbindungsgruppen d, e, f, g, h, i, k, l, m, n, o, p, r, s, t, u, und v bilden.

Die Herstellung neuer Verbindungen der Formel I erfolgt, indem man eine Verbindung der Formel II

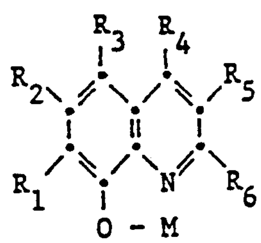

(II),

worin $R_1$, $R_2$, $R_3$, $R_4$, $R_5$ und $R_6$ die für Formel I angegebenen Bedeutungen haben und M für Wasserstoff, ein Alkali- oder Erdalkalimetallatom steht, mit einer Verbindung der Formel III

Z—X—Y (III)

worin X und Y die für Formel I angegebenen Bedeutungen haben und Z für einen abspaltbaren Rest steht, umsetzt.

In der Verbindung der formel III kommt als abspaltbarer Rest Z insbesondere ein Halogenatom oder eine Methylsulfonyloxy-, Phenylsulfonyloxy- oder para-Tolylsulfonyloxygruppe in Betracht. Halogen steht hier für Fluor, Chlor, Brom und Jod, bevorzugt für chlor und Brom.

Wenn in der Verbindung der Formel II M für Wasserstoff und in der Verbindung der Formel III Z für ein Halogenatom steht, so lässt sich die Umsetzung bevorzugt in Gegenwart eines üblichen Protonenakzeptors durchführen. Ferner wirkt, wenn in der Verbindung der Formel III Z für ein Halogenatom steht, die Zugabe einer geringen Menge Alkalijodid katalytisch.

Die Umsetzung wird zweckmässigerweise in Gegenwart von gegenüber den Reaktionssteilnehmern inerten Lösungsmitteln durchgeführt. Als solche kommen beispielsweise Kohlenwasserstoffe wie Benzol, Toluol, Xylol, Petroläther oder Cyclohexan, Aether wie beispielsweise Diäthyläther, Dipropyläther, Tetrahydrofuran, Dioxan, Dimethoxyäthan oder Diäthylenglykol-dimethyläther, Säureamide wie beispielsweise Dimethylformamid, Dimethylacetamid, 2-Pyrrolidinon oder Hexamethylphosphorsäuretriamid oder Sulfoxide wie beispielsweise Dimethylsulfoxid in Betracht.

Als säurebindende Mittel können beispielsweise Alkalimetall- und Erdalkalimetallhydroxide oder -alkoholate, Alkalicarbonate oder tertiäre organische Basen eingesetzt werden.

Die Reaktionstemperaturen liegen im allgemeinen in einem Bereich von 0 bis 200°C, insbesondere im Bereich von 50 bis 150°C.

Die verwendeten Ausgangsprodukte sind bekannt oder können analog bekannten Verfahren hergestellt werden.

Auf diese Weise lassen sich beispielsweise Verbindungen von als bevorzugt angegebenen Gruppen wie folgt herstellen:

Verbindungen der Gruppe g) erhält man, indem man eine Verbindung der Formel IV

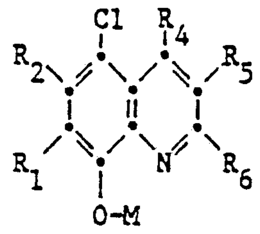

(IV)

worin $R_1$, $R_2$, $R_4$, $R_5$ und $R_6$ die für die Formel I angegebene Bedeutung haben und M für Wasserstoff, ein Alkali-oder Erdalkaliatom steht, mit $Z—CH_2—CH=CH—CH_3$, worin Z für einen abspaltbaren Rest steht, umsetzt.

Verbindungen der Gruppe h) erhält man, indem man eine Verbindung der Formel V

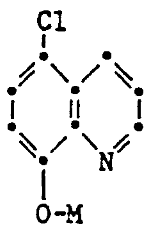

(V)

worin M für Wasserstoff, ein Alkali-oder Erdalkaliatom steht, mit $Z—(CH_2)_7—CH_3$, worin Z für einen abspaltbaren Rest steht, umsetzt.

Verbindungen der Gruppe i) erhält man, indem man eine Verbindung der Formel V

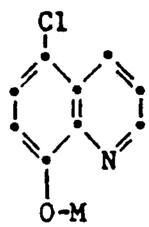

(V)

worin M für Wasserstoff, ein Alkali- oder Erdalkaliatom steht, mit $Z—CH_2—CH=CH—CH_3$, worin Z für einen abspaltbaren Rest steht, umsetzt.

Verbindungen der Gruppe n) erhält man, indem man eine Verbindung der Formel V

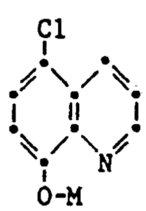

(V)

worin M für Wasserstoff, ein Alkali- oder Erdalkaliatom steht, mit

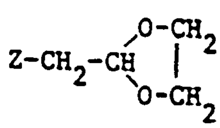,

worin Z für einen abspaltbaren Rest steht, umsetzt.

Verbindungen der Gruppe o) erhält man, indem man eine Verbindung der Formel V

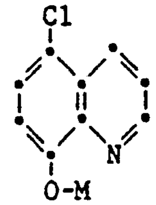

(V)

worin M für Wasserstoff, ein Alkali- oder Erdalkaliatom steht, mit

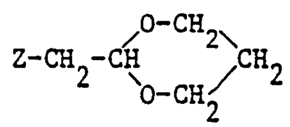

,

worin Z für einen abspaltbaren Rest steht, umsetzt.

Verbindungen der Gruppe p) erhält man, indem man eine Verbindung der Formel V

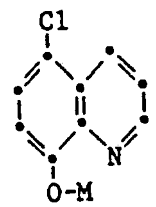

(V)

worin M für Wasserstoff, ein Alkali- oder Erdalkaliatom steht, mit

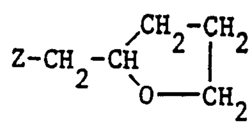

,

worin Z für einen abspaltbaren Rest steht, umsetzt.

Verbindungen der Gruppe r) erhält man, indem man eine Verbindung der Formel VI

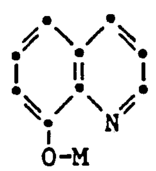

(VI)

worin M für Wasserstoff, ein Alkali- oder Erdalkaliatom steht, mit $Z—CH_2—CH=CH—CH_3$, worin Z für einen abspaltbaren Rest steht, umsetzt.

Verbindungen der Gruppe t) erhält man, indem man eine Verbindung der Formel VI

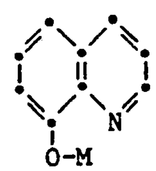

(VI)

worin M für Wasserstoff, ein Alkali- oder Erdalkaliatom steht, mit

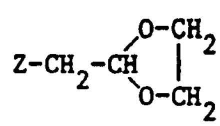

,

worin Z für einen abspaltbaren Rest steht, umsetzt.

Verbindungen der Gruppe u) erhält man, indem man eine Verbindung der Formel VI

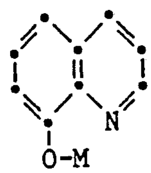

(VI)

worin M für Wasserstoff, ein Alkali- oder Erdalkaliatom steht, mit

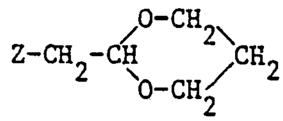

,

worin Z für einen abspaltbaren Rest steht, umsetzt.

Verbindungen der Gruppe v) erhält man, indem man eine Verbindung der Formel VI

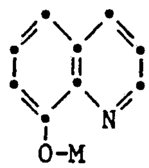

(VI)

worin M für Wasserstoff, ein Alkali- oder Erdalkaliatom steht, mit

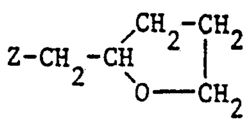

,

worin Z für einen abspaltbaren Rest steht, umsetzt.

Die bereits bekannten Chinolinderivate können analog der vorstehend beschriebenen Verfahrensweise oder gemäss einem der in der nachfolgend genannten Literatur beschriebenen Verfahren zur Herstellung von 8-Alkoxychinolinen hergestellt werden: J. of General Chemistry of USSR 1952, Vol *22*, S. 1263—1266; Acta Pharmaceutica Internationale Vol II, No. 1—5, p 149—161; Arch. der Pharmazie *279* (1941), p. 154—165; Bl (5) *12* (1945), p. 866—870; J. Am. Chem. Soc. *52*, p. 4433 (1930); Helv. Chim. acta *27*, p. 1736; Ber. *14*, 2570; Ber. *49*, 518; J.p. Chem. *93*, 376.

Die Verbindungen lassen sich auch nach einem in jüngerer Zeit bekanntgewordenen Herstellungsverfahren, dem Phasen-Transfer-Catalytischen Verfahren von Chin-Hsien et al aus Synthesis 1982, Nr. 10, p. 858—861 herstellen.

Die als Ausgangsstoffe verwendeten 8-Oxychinoline und ihre Substitutionsprodukte sind bekannt: 8-Oxychinolin, 8-Oxychinaldin, 5-Chlor-8-oxychinolin, 5-Chlor-8-oxychinaldin, 7-Chlor-8-oxychinolin, 7-Chlor-8-oxychinaldin, 5,7-Dichlor-8-oxychinolin, 5-Chlor-7-jod-8-oxychinolin, 5-Brom-8-oxychinolin, 5,7-Dibromchinolin und weitere. Ihre Herstellung findet sich teilweise in Beta Pharmaceutica Internationale Vol II Nr. 1—5, p. 153 (1951) Copenhagen. Die Herstellung von 5-Aceto-8-oxychinolin, 5-Aethyl-8-oxychinolin wird in Arch. der Pharmazie *279* (1941) S. 154 beschrieben.

Bevorzugte Ausgangsstoffe sind das 8-Oxychinolin, das 8-Oxychinaldin, das 5-Chlor-8-Oxychinolin, und das 5-Chlor-8-oxychinaldin.

Herstellungsbeispiele für Wirkstoffe:

Beispiel 1

5-Chlor-8-(2-butenyloxy)-chinolin (Verbindung Nr. 1)

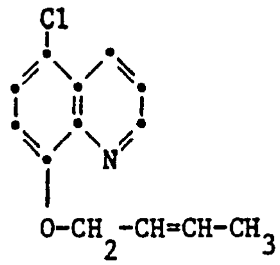

3,6 g (0,09 Mol) NaOH-Plätzchen werden in 300 ml Wasser gelöst, 300 ml Methylenchlorid zugegeben, 10,8 g (0,06 Mol) 5-Chlor-8-oxychinolin und 16,2 g 2-Butenylbromid (0,12 Mol) und 0,5 g Tetra-n-butylammoniumbromid zugefügt und 5 Stunden bei Zimmertemperatur gerührt. Die organische Schicht wird abgetrennt, und die wässrige Schicht zweimal mit Methylenchlorid extrahiert. Die organischen Lösungsmittel werden vereinigt und eingedampft. Der Rückstand wird in Wasser und Methylenchlorid aufgenommen, die organische Schicht dreimal mit 2-n NaOH ausgeschüttelt, getrennt, mit $Na_2SO_4$ gerocknet und eingedampft. Rohausbeute 15,5 g.

Dest. bei 0,08—0,06 mbar, 122—120°C: 8,9 g, Sdp. 123—131/0.06—0.08 $C_{13}H_{12}Cl$ NO Mg 233,5

| | | |
|---|---|---|
| Ber.: | 66,81% C | Gef. 67,0% C |
| | 5,18% H | 5,6% H |
| | 6,0% N | 5,6% N |

Beispiel 2

8-(1,3-Dioxolan-2-ylmethoxy)-chinolin (Verbindung Nr. 2)

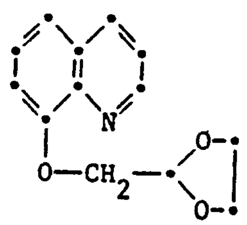

36,6 g Kalium-8-chinolinoxid werden fein gemahlen und in 120 ml Dimethylsulfoxid auf 45—50° unter Rühren erwärmt. Nachdem alles gelöst ist, wird wieder abgekühlt und bei Raumtemperatur mit 33,4 g 2-Brommethyl-1,3-dioxolan versetzt. Es wird während 6 Stunden auf 60—65° gerührt und anschliessend eingedampft. Der Rückstand wird in Wasser und Methylenchlorid aufgenommen, die organische Schicht über Kieselerde filtriert und anschliessend dreimal mit Wasser und verdünnter NaOH gewaschen, dann mit Wasser neutral gewaschen, mit $Na_2SO_4$ getrocknet und eingedampft.

Dest. bei 0,1 mbar, 145—150°C: Smp. 60—63°, 12,9 g. $C_{13}H_{13}O_3N$ Mg 231,25

| | | |
|---|---|---|
| Ber.: | 67,52% C | Gef. 67,2% C |
| | 5,67% H | 5,9% H |
| | 6,06% N | 6,4% N |

In gleicher Weise wird das 5-Chlor-8-(1,3-dioxolan-2-ylmethoxy-chinolin

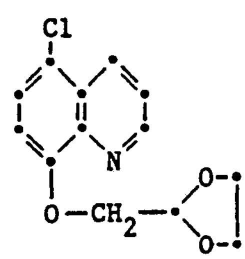

(Verbindung Nr. 3)

hergestellt.

Analog einer der vorstehend beschriebenen Methoden lassen sich auch die folgenden, in der Tabelle 1 zusammen mit den Verbindungen der vorstehenden Beispiele aufgeführten Verbindungen der Formel I herstellen:

Tabelle 1

| Verb. Nr. | $R_1$ | $R_2$ | $R_3$ | $R_4$ | $R_5$ | $R_6$ | X | Y | Physikal. Konstante |
|---|---|---|---|---|---|---|---|---|---|
| 1 | H | H | Cl | H | H | H | $-CH_2-CH=CH-CH_2-$ | H | Sdp. 123-127°C/ 0.06-0.08 mm |
| 2 | H | H | H | H | H | H | $-CH_2-$ | 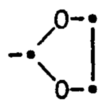 | Smp. 60 - 63°C |
| 3 | H | H | Cl | H | H | H | $-CH_2-$ | 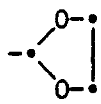 | |
| 4 | H | H | H | H | H | H | $-CH_2-CH=CH-CH_2-$ | H | Sdp. 107-110°C/0.06 mm |
| 5 | H | H | H | H | H | H | $-(CH_2)_8-$ | H | Sdp.163-164°C 6,8 mm |
| 6 | H | H | Cl | H | H | H | $-(CH_2)_8-$ | H | |
| 7 | H | H | H | H | H | $CH_3$ | $-CH_2-CH=CH-$ | H | |
| 8 | H | H | H | H | H | $CH_3$ | $-(CH_2)_8-$ | H | Oel |
| 9 | H | H | H | H | H | H | $-CH_2-$ | 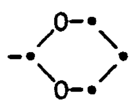 | |
| 10 | H | H | Cl | H | H | H | $-CH_2-$ | 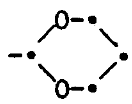 | |

Tabelle 1 (Fortsetzung)

| Verb. Nr. | $R_1$ | $R_2$ | $R_3$ | $R_4$ | $R_5$ | $R_6$ | X | Y | Physikal. Konstante |
|---|---|---|---|---|---|---|---|---|---|
| 11 | Cl | H | Cl | H | H | H | $-(CH_2)_8-$ | H | Smp. 41-43°C |
| 12 | Br | H | Br | H | H | H | $-(CH_2)_8-$ | H | |
| 13 | H | H | $NO_2$ | H | H | H | $-(CH_2)_4-$ | H | Smp. 107-108,5°C |
| 14 | Br | H | Br | H | H | H | $-(CH_2)_4-$ | H | Smp. 61-63°C |
| 15 | H | H | Cl | H | H | H | $-CH_2-$ | 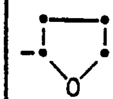 | |
| 16 | Cl | H | Cl | H | H | H | $-CH_2-$ | 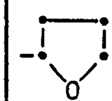 | |
| 17 | H | H | Cl | H | H | H | $-CH_2-$ | 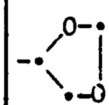 | |
| 18 | H | H | Cl | H | H | H | $-CH_2-$ | 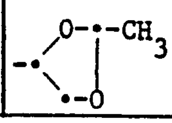 | |

Tabelle 1 (Fortsetzung)

| Verb. Nr. | $R_1$ | $R_2$ | $R_3$ | $R_4$ | $R_5$ | $R_6$ | X | Y | Physikal. Konstante |
|---|---|---|---|---|---|---|---|---|---|
| 19 | H | H | Cl | H | H | H | $-CH-CH_2-CH_2-$<br>$CH_3$ | H | |
| 20 | H | H | H | H | H | $CH_3$ | $-CH_2-$ | 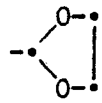 | |
| 21 | H | H | Cl | H | H | $CH_3$ | $-CH_2-$ | 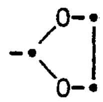 | |
| 22 | H | H | Cl | H | H | $CH_3$ | $-CH_2-CH=CH-CH_2-$ | H | |

Tabelle 1 (Fortsetzung)

| Verb. Nr. | $R_1$ | $R_2$ | $R_3$ | $R_4$ | $R_5$ | $R_6$ | X | Y | Physikal. Konstante |
|---|---|---|---|---|---|---|---|---|---|
| 23 | Cl | H | H | H | H | $CH_3$ | $-CH_2-CH=CH-CH_2-$ | H | |
| 24 | Cl | H | Cl | H | H | H | $-CH_2-$ | 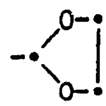 | |
| 25 | Cl | H | Cl | H | H | H | $-CH_2-CH=CH-CH_2-$ | H | |
| 26 | H | H | H | H | H | H | $-CH_2-CH=CH-$ | H | |
| 27 | H | H | Cl | H | H | H | $-CH_2-CH=CH-$ | H | |
| 28 | H | H | Cl | H | H | H | $-CH_2-C\equiv C-$ | H | |
| 29 | H | H | Cl | H | H | $CH_3$ | $-(CH_2)_8-$ | H | |

Formulierungsbeispiele für flüssige Wirkstoffe der Formel I (% = Gewichtsprozent)

| 3. Emulsions-Konzentrate | a) | b) | c) |
|---|---|---|---|
| Wirkstoff aus Tabelle 1 | 25% | 40% | 50% |
| Ca-Dodecylbenzolsulfonat | 5% | 8% | 6% |
| Ricinusöl-polyäthylenglykoläther (36 Mol AeO) | 5% | — | — |
| Tributylphenol-polyäthylenglykoläther (30 Mol Aeo) | — | 12% | 4% |
| Cyclohexanon | — | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 4. Lösungen | a) | b) | c) | d) |
|---|---|---|---|---|
| Wirkstoff aus Tabelle 1 | 80% | 10% | 5% | 95% |
| Aethylenglykol-monomethyl-äther | 20% | — | — | — |
| Polyäthylenglykol M G 400 | — | 70% | — | — |
| N-Methyl-2-pyrrolidon | — | 20% | — | — |
| Epoxydiertes Kokosnussöl | — | — | 1% | 5% |
| Benzin (Siedegrenzen 160—190°C) | — | — | 94% | — |

Die Lösungen sind zur Anwendung in Form kleinster Tropfen geeignet.

5. Granulate

| | a) | b) |
|---|---|---|
| Wirkstoff aus Tabelle 1 | 5% | 10% |
| Kaolin | 94% | — |
| Hochdisperse Kieselsäure | 1% | — |
| Attapulgit | — | 90% |

Der Wirkstoff wird in Methylenchlorid gelöst, auf den Träger aufgesprüht und das Lösungsmittel anschliessend im Vakuum abgedampft.

6. Stäubemittel

| | a) | b) |
|---|---|---|
| Wirkstoff aus Tabelle 1 | 2% | 5% |
| Hochdisperse Kieselsäure | 1% | 5% |
| Talkum | 97% | — |
| Kaolin | — | 90% |

Durch inniges Vermischen der Trägerstoffe mit dem Wirkstoff erhält man gebrauchsfertige Stäubemittel.

Formulierungsbeispiele für feste Wirkstoffe der Formel I
(% = Gewichtsprozent)

7. Spritzpulver

| | a) | b) | c) |
|---|---|---|---|
| Wirkstoff aus Tabelle 1 | 25% | 50% | 75% |
| Na-Ligninsulfonat | 5% | 5% | — |
| Na-Laurylsulfat | 3% | — | 5% |
| Na-Diisobutylnaphthalinsulfonat | — | 6% | 10% |
| Octylphenolpolyäthylenglykoläther (7—8 mol Aeo) | — | 2% | — |
| Hochdisperse Kieselsäure | 5% | 10% | 10% |
| Kaolin | 62% | 27% | — |

Der Wirkstoff wird mit den Zusatzstoffen gut vermischt und in einer geeigneten Mühle gut vermahlen. Man erhält Spritzpulver, die sich mit Wasser zu Suspensionen Jeder gewünschten Konzentration verdünnen lassen.

8. Emulsions-Konzentrat

| | |
|---|---|
| Wirkstoff aus Tabelle 1 | 10% |
| Octylphenolpolyäthylenglykoläther (4—5 Mol AeO) | 3% |
| Ca-Dodecylbenzolsulfonat | 3% |
| Ricinusölpolyglykoläther (35 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

9. Stäubemittel

| | a) | b) |
|---|---|---|
| Wirkstoff aus Tabelle 1 | 5% | 8% |
| Talkum | 95% | — |
| Kaolin | — | 92% |

Man erhält anwendungsfertige Stäubemittel, indem der Wirkstoff mit den Trägerstoffen vermischt und auf einer geeigneten Mühle vermahlen wird.

10. Extruder Granulat

| | |
|---|---|
| Wirkstoff aus Tabelle 1 | 10% |
| Na-Ligninsulfonat | 2% |
| Carboxymethylcellulose | 1% |
| Kaolin | 87% |

Der Wirkstoff wird mit den zusatzstoffen vermischt, vermahlen und mit Wasser angefeuchtet. Dieses Gemisch wird extrudiert und anschliessend im Luftstrom getrocknet.

11. Umhüllungs-Granulat

| | |
|---|---|
| Wirkstoff aus Tabelle 1 | 3% |
| Polyäthylenglykol (M G 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Wiese erhält man staubfreie Umhüllungs-Granulate.

12. Suspensions-Konzentrat

| | |
|---|---|
| Wirkstoff aus Tabelle 1 | 40% |
| Aethylenglykol | 10% |
| Nonylphenolpolyäthylenglykoläther (15 Mol AeO) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Formulierungsbeispiele für Wirkstoffgemische (flüssig (% = Gewichtsprozent)

| 13. Emulsions-Konzentrate | a) | b) | c) |
|---|---|---|---|
| Wirkstoffgemisch: Antidot aus Tabelle 1 und ein Herbizid im Verhältnis 1:1 | 25% | 40% | 50% |
| Ca-Dodecylbenzolsulfonat | 5% | 8% | 6% |
| Ricinusöl-polyethylenglykolether (36 Mol AeO) | 5% | — | — |
| Tributylphenol-polyethylenglykolether (30 Mol AeO) | — | 12% | 4% |
| Cylcohexanon | — | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 14. Emulsions-Konzentrate | a) | b) | c) |
|---|---|---|---|
| Wirkstoffgemisch: Antidot aud Tabelle 1 und ein Herbizid im Verhältnis 1:3 | 25% | 40% | 50% |
| Ca-Dodecylbenzolsulfonat | 5% | 8% | 6% |
| Ricinusöl-polyethylenglykolether (36 Mol AeO) | 5% | — | — |
| Tributylphenol-polyethylenglykolether (30 Mol AeO) | — | 12% | 4% |
| Cyclohexanon | — | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 15. Emulsions-Konzentrate | a) | b) | c) |
|---|---|---|---|
| Wirkstoffgemisch: Antidot aus Tabelle 1 und ein Herbizid im Verhältnis 2:1 | 25% | 40% | 50% |
| Ca-Dodecylbenzolsulfonat | 5% | 8% | 6% |
| Ricinusöl-polyethylenglykolether (36 Mol AeO) | 5% | — | — |
| Tributylphenol-polyethylenglykolether (30 Mol AeO) | — | 12% | 4% |
| Cyclohexanon | — | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 16. Emulsions-Konzentrate | a) | b) | c) |
|---|---|---|---|
| Wirkstoffgemisch: Antidot aud Tabelle 1 und 2-[4-(3,5-Dichlorpyridyl-2-oxy)-phenoxy]-propionsäure-2-propinylester im Verhältnis 1:1 | 25% | 40% | 50% |
| Ca-Dodecylbenzosulfonat | 5% | 8% | 6% |
| Ricinusöl-polyethylenglykolether (36 Mol AeO) | 5% | — | — |
| Tributylphenol-polyethylenglykolether (30 Mol AeO) | — | 12% | 4% |
| Cyclohexanon | — | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 17. Emulsions-Konzentrat | a) | b) | c) |
|---|---|---|---|
| Wirkstoffgemisch: Antidot aus Tabelle 1 und 2-[4-(3,5-Dichlorpyridyl-2-oxy)-phenoxy]-propionsäure-2-propinylester im Verhältnis 1:3 | 25% | 40% | 50% |
| Ca-Dodecylbenzolsulfonat | 5% | 8% | 6% |
| Ricinusöl-polyethylenglykolether (36 Mol AeO) | 5% | — | — |
| Tributylphenol-polyethylenglykolether (30 Mol AeO) | — | 12% | 4% |
| Cyclohexanon | — | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 18. Lösungen | a) | b) | c) | d) |
|---|---|---|---|---|
| Wirkstoffgemisch: Antidot aus Tabelle 1 und eine Herbizid im Verhältnis 1:4 | 80% | 10% | 5% | 95% |
| Ethylenglykol-monomethyl-ether | 20% | — | — | — |
| Polyethylenglykol MG 400 | — | 70% | — | — |
| N-Methyl-2-pyrrolidon | — | 20% | — | — |
| Epoxydiertes Kokosnussöl | — | — | 1% | 5% |
| Benzin (Siedegrenzen 160—190°C) | — | — | 94% | — |

Die Lösungen sind zur Anwendung in Form kleinster Tropfen geeignet.

19. Lösungen

| | a) | b) | c) | d) |
|---|---|---|---|---|
| Wirkstoffgemisch: Antidot aus Tabelle 1 und ein Herbizid im Verhältnis 5:2 | 80% | 10% | 5% | 95% |
| Ethylenglykol-monomethyl-ether | 20% | — | — | — |
| Polyethylenglykol MG 400 | — | 70% | — | — |
| N Methyl-2-pyrrolidon | — | 20% | — | — |
| Epoxydiertes Kokosnussöl | — | — | 1% | 5% |
| Benzin (Siedegrenzen 160—190°C) | — | — | 94% | — |

Die Lösungen sind zur Anwendung in Form kleinster Tropfen geeignet.

20. Lösungen

| | a) | b) | c) | d) |
|---|---|---|---|---|
| Wirkstoffgemisch Antidot aus Tabelle 1 und ein Herbizid im Verhältnis 1:1 | 80% | 10% | 5% | 95% |
| Ethylenglykol-monomethyl-ether | 20% | — | — | — |
| Polyethylenglykol MG 400 | — | 70% | — | — |
| N-Methyl-2-pyrrolidon | — | 20% | — | — |
| Epoxydiertes Kokosnussöl | — | — | 1% | 5% |
| Benzin (Siedegrenzen 160—190°C) | — | — | 94% | — |

Die Lösungen sind zur Anwendung in Form kleinster Tropfen geeignet.

21. Lösungen

| | a) | b) | c) | d) |
|---|---|---|---|---|
| Wirkstoffgemisch: Antidot aus Tabelle 1 und 2-[4-(3,5-Dichlorpyridyl-2-oxy)-phenoxy]-propionsäure-2-propinylester im Verhältnis 1:1 | 80% | 10% | 5% | 95% |
| Ethylenglykol-monomethyl-ether | 20% | — | — | — |
| Polyethylenglykol MG 400 | — | 70% | — | — |
| N-Methyl-2-pyrrolidon | — | 20% | — | — |
| Epoxydiertes Kokosnussöl | — | — | 1% | 5% |
| Benzin (Siedegrenzen 160—190°C) | — | — | 94% | — |

Die Lösungen sind zur Anwendung in Form kleinster Tropfen geeignet.

| 22. Lösungen | a) | b) | c) | d) |
|---|---|---|---|---|
| Wirkstoffgemisch: Antidot aus Tabelle 1 und 2-[4-(3,5-Dichlorpyridyl-2-oxy)-phenoxy]-propionsäure-2-propinylester im Verhältnis 1:4 | 80% | 10% | 5% | 95% |
| Ethylenglykol-monomethyl-ether | 20% | — | — | — |
| Polyethylenglykol MG 400 | — | 70% | — | — |
| N-Methyl-2-pyrrolidon | — | 20% | — | — |
| Epoxydiertes Kokosnussöl | — | — | 1% | 5% |
| Benzin (Siedegrenzen 160—190°C) | — | — | 94% | — |

Die Lösungen sind zur Anwendung in Form kleinster Tropfen geeignet.

| 23. Granulate | a) | b) |
|---|---|---|
| Wirkstoffgemisch: Antidot aus Tabelle 1 und ein Herbizid im Verhältnis 1:1 | 5% | 10% |
| Kaolin | 94% | — |
| Hochdisperse Kieselsäure | 1% | — |
| Attapulgit | — | 90% |

Der Wirkstoff wird in Methylenchlorid gelöst, auf den Träger aufgesprüht und das Lösungsmittel anschliessend in Vakuum abgedampft.

| 24. Granulate | a) | b) |
|---|---|---|
| Wirkstoffgemisch: Antidot aus Tabelle 1 und 2-[4-(3,5-Dichlorpyridyl-2-oxy)-phenoxy]-propionsäure-2-propinylester im Verhältnis 1:1 | 5% | 10% |
| Kaolin | 94% | — |
| Hochdisperse Kieselsäure | 1% | — |
| Attapulgit | — | 90% |

Der Wirkstoff wird im Methylenchlorid gelöst, auf den Träger aufgesprüht und das Lösungsmittel anschliessend im Vakuum abgedampft.

| 25. Stäubemittel | a) | b) |
|---|---|---|
| Wirkstoffgemisch: Antidot aus Tabelle 1 und ein Herbizid im Verhältnis 1:1 | 2% | 5% |
| Hochdisperse Kieselsäure | 1% | 5% |
| Talkum | 97% | — |
| Kaolin | — | 90% |

Durch inniges Vermischen der Trägerstoffe mit dem Wirkstoff erhält man gebrauchsfertige Stäubemitel.

Formulierungsbeispiele für Wirkstoffgemische (fest)
(% = Gewichtsprozent)

| 26. Spritzpulver | a) | b) | c) |
|---|---|---|---|
| Wirkstoffgemisch: Antidot aus Tabelle 1 und ein Herbizid im Verhältnis 1:1 | 25% | 50% | 75% |
| Na-Ligninsulfonat | 5% | 5% | — |
| Na-Laurylsulfat | 3% | — | 5% |
| Na-Diisobutylnaphthalinsulfonat | — | 6% | 10% |
| Octylphenolpolyethylenglykolether (7—8 Mol AeO) | — | 2% | — |
| Hochdisperse Kieselsäure | 5% | 10% | 10% |
| Kaolin | 62% | 27% | — |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 27. Spritzpulver | a) | b) | c) |
|---|---|---|---|
| Wirkstoffgemisch: Antidot aus Tabelle 1 und ein Herbizid im Verhältnis 1:4 | 25% | 50% | 75% |
| Na-Ligninsulfonat | 5% | 5% | — |
| Na-Laurylsulfat | 3% | — | 5% |
| Na-Diisobutylnaphthalinsulfonat | — | 6% | 10% |
| Octylphenolpolyethylenglykolether (7—8 Mol AeO) | — | 2% | — |
| Hochdisperse Kieselsäure | 5% | 10% | 10% |
| Kaolin | 62% | 27% | — |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 28. Spritzpulver | a) | b) | c) |
|---|---|---|---|
| Wirkstoffgemisch: Antidot aus Tabelle 1 und ein Herbizid im Verhältnis 3:1 | 25% | 50% | 75% |
| Na-Ligninsulfonat | 5% | 5% | — |
| Na-Laurylsulfat | 3% | — | 5% |
| Na-Diisobutylnaphthalinsulfonat | — | 6% | 10% |
| Octylphenolpolyethylenglykolether (7—8 Mol AeO) | — | 2% | — |
| Hochdisperse Kieselsäure | 5% | 10% | 10% |
| Kaolin | 62% | 27% | — |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

29. Emulsions-Konzentrate

| | |
|---|---|
| Wirkstoffgemisch: Antidot aus Tabelle 1 und ein Herbizid im Verhältnis 1:1 | 10% |
| Octylphenolpolyethylenglykolether (4—5 Mol AeO) | 3% |
| Ca-Dodecylbenzolsulfonat | 3% |
| Ricinusölpolyglykolether (35 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

30. Emulsions-Konzentrate

| | |
|---|---|
| Wirkstoffgemisch: Antidot aus Tabelle 1 und ein Herbizid im Verhältnis 5:2 | 10% |
| Octylphenolpolyethylenglykolether (4—5 Mol AeO) | 3% |
| Ca-Dodecylbenzolsulfonat | 3% |
| Ricinusölpolyglykolether (35 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

31. Emulsions-Konzentrate

| | |
|---|---|
| Wirkstoffgemisch: Antidot aus Tabelle 1 und ein Herbizid im Verhältnis 1:4 | 10% |
| Octylphenolpolyethylenglykolether (4—5 Mol AeO) | 3% |
| Ca-Dodecylbenzolsulfonat | 3% |
| Ricinusölpolyglykolether (35 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

32. Stäubemittel

| | a) | b) |
|---|---|---|
| Wirkstoffgemisch: Antidot aus Tabelle 1 und ein Herbizid im Verhältnis 1:1 | 5% | 8% |
| Talkum | 95% | — |
| Kaolin | — | 92% |

Man erhält anwendungsfertige Stäubemittel, indem der Wirkstoff mit den Trägerstoffen vermischt und auf einer geeigneten Mühle vermahlen wird.

33. Extruder-Granulate

| | |
|---|---|
| Wirkstoffgemisch: Antidot aus Tabelle 1 und ein Herbizid im Verhältnis 1:1 | 10% |
| Na-Liginsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

34. Umhüllungs-Granulate

| | |
|---|---|
| Wirkstoffgemisch: Antidot aus Tabelle 1 und ein Herbizid im Verhältnis 1:1 | 3% |
| Polyethylenglykol (MG 200) | 3% |
| Kaolin | 94% |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

35. Suspensions-Konzentrate

| | |
|---|---|
| Wirkstoffgemisch: Antidot aus Tabelle 1 und ein Herbizid im Verhältnis 1:1 | 40% |
| Ethylenglykol | 10% |
| Nonylphenolpolyethylenglykolether (15 Mol AeO) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

36. Suspensions-Konzentrate

| | |
|---|---|
| Wirkstoffgemisch: Antidot aus Tabelle 1 und ein Herbizid im Verhältnis 1:4 | 40% |
| Ethylenglykol | 10% |
| Nonylphenolpolyethylenglykolether (15 Mol AeO) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

37. Suspensions-Konzentrate

| | |
|---|---|
| Wirkstoffgemisch: Antidot aus Tabelle 1 und ein Herbizid im Verhältnis 3:1 | 40% |
| Ethylenglykol | 10% |
| Nonylphenolpolyethylenglykolether (15 Mol AeO) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologiche Beispiele

Beispiel 38

Versuch mit Antidot und Herbizid an Weizen

Weizensamen werden in Plastiktöpfe, die 0,5 l Gartenerde enthalten, im Gewächshaus ausgesät. Nach dem Auflaufen der Pflanzen bis zum 2- bis 3-Blattstadium wird die als Antidot zu prüfende Substanz zusammen mit dem Herbizid 2-[4-3,5-Dichlorpyridyl-2-oxy)-phenoxy]-propionsäure-2-propinylester als Tankmischung appliziert. Die Aufwandmengen an Antidot und Herbizid entsprechen je 0,5 kg AS/ha. 20 Tage nach der applikation wird die Schutzwirkung des Antidots in Prozent bonitiert. Als Referenzen dienen dabei mit dem Herbizid allein behandelte Pflanzen sowie vollständig unbehandelte Kontrollpflanzen. Die Ergebnisse zeigt die folgende Tabelle:

Tabelle 2

| Verbindung Nr. | relative Schutzwirkung in Prozent |
|---|---|
| 1 | 50 |
| 3 | 50 |

Beispiel 39

Nachauflaufapplikation von Herbizid und Safener in Tankmischung

Plastiktöpfe (oberer Duchmesser 7 cm) werden mit sandig-toniger Lehmerde gefüllt. Weizensamen der Sorte "Besso" werden eingesät, mit Erde bedeckt und anschliessend angegossen. Die Pflanzen werden im Gewächshaus bis zum 2- bis 3-Blattstadium kultiviert. Dann wird die als Safener zu prüfende Substanz in Wasser gelöst und zusammen mit dem Herbizid 2-[4-(3,5-Dichlorpyridyl-2-oxy)phenoxy]-propionsäure-2-propinylester in insgesamt 550 l Brühe/ha auf die Pflanzen gesprüht. Die Aufwandmenge an Safener entspricht 1,5 kg AS/ha, an Herbizid 0,75 kg AS/ha. Drei Wochen nach der Applikation wird die Schutzwirkung des Safeners in Prozent bonitiert. Als Referenz dienen dabei mit dem Herbizid allein behandelte Pflanzen sowie vollständig unbehandelte Kontrolpflanzen. Die Resultate sind in der nachfolgenden Tabelle dargestellt.

Tabelle 3

| Verbindung Nr. | Relative Schutzwirkung in Prozent |
|---|---|
| 1 | 75 |
| 2 | 63 |
| 4 | 38 |
| 5 | 38 |
| 8 | 38 |

Beispiel 40

Nachauflaufapplikation von Herbizid und Safener in Tankmischung

Plastiktöpfe (oberer Durchmesser 11 cm, Inhalt 500 $cm^3$) werden mit sandig-tonigert Lehmerde gefüllt. Weizensamen der Sorte "Besso" werden eingesät, mit Erde bedeckt und anschliessend angegossen. Die Pflanzen werden im Gewächshaus bis zum 2- bis 3-Blattstadium kultiviert. Dann wird die als Safener zu prüfende Substanz in Wasser gelöst und zusammen mit dem Herbizid 2-[4-(3,5-Dichlorpyridyl-2-oxy)-phenoxy]-propionsäure-2-propinylester in insgesamt 550 l Brühe/ha auf die Pflanzen gesprüht. Drei Wochen nach der Applikation wird die Schutzwirkung des Safeners in Prozent bonitiert. Als Referenz dienen dabei mit dem Herbizid allein behandelte Pflanzen sowie vollständig unbehandelte Kontrollpflanzen. Die Resultate sind in der nachfolgenden Tabelle dargestellt.

Tabelle 4

| Verbindung Nr. | Safener kg AS/ha | Herbizid kg AS/ha | Relative Schutzwirkung in Prozent |
|---|---|---|---|
| 1 | 1,0 | 1,0 | 75 |
| 1 | 0,5 | 1,0 | 75 |
| 1 | 0,75 | 0,75 | 75 |
| 1 | 0,38 | 0,75 | 75 |
| 1 | 0,5 | 0,5 | 75 |
| 1 | 0,25 | 0,5 | 62,5 |
| 2 | 1,0 | 1,0 | 62,5 |
| 2 | 0,5 | 1,0 | 62,5 |
| 2 | 0,75 | 0,75 | 37,5 |
| 2 | 0,38 | 0,75 | 37,5 |
| 2 | 0,5 | 0,5 | 12,5 |
| 2 | 0,25 | 0,5 | 25 |

Beispiel 41

Nachauflaufapplikation von Herbizid und Safener in Tankmischung

Plastikbehälter (Länge × Breite × Höhe = 25 × 17 × 21 cm) werden mit sandig-toniger Lehmerde gefüllt. Weizensamen der Sorte "Besso" werden eingesät, mit Erde bedeckt und anschliessend angegossen. Die Pflanzen werden im Gewächshaus bis zum 2- bis 3-Blattstadium kultiviert. Dann wird das als Safener zu prüfende 5-Chlor-8-(2-butenyloxy)chinolin (Verbindung Nr. 1) in Wasser gelöst und zusammen mit dem Herbizid 2-[4-(3,5-Dichlorpyridyl-2-oxy)-phenoxy]-propionsäure-2-propinylester in insgesamt 550 l Brühe/ha auf die Pflanzen gesprüht. Drei Wochen nach der Applikation wird die Schutzwirkung des Safeners in Prozent bonitiert. Als Referenz dienen dabei mit dem Herbizid allein behandelte Pflanzen sowie vollständig unbehandelte Kontrollpflanzen. Die Resultate zeigt die nachfolgenden Tabelle.

Tabelle 5

| Safener kg AS/ha | Herbizid kg AS/ha | Relative Schutzwirkung in Prozent |
|---|---|---|
| 1,5 | 1,5 | 62,5 |
| 1,5 | 0,75 | 62,5 |
| 1,5 | 0,375 | 62,5 |
| 1,0 | 1,0 | 50 |
| 1,0 | 1,5 | 50 |
| 1,0 | 0,25 | 50 |

**Patentansprüche**

1. Verfahren zum Schützen von Kulturpflanzen gegen schädigende Wirkungen von Herbiziden, dadurch gekennzeichnet, dass man die Kulturpflanzen, Teile dieser Pflanzen oder Anbauflächen für Kulturpflanzen mit einer Verbindung der Formel I

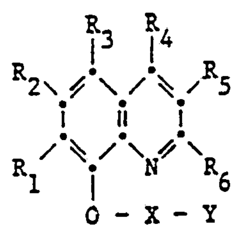

(I),

worin

$R_1$, $R_2$ und $R_3$ unabhangig voneinander Wasserstoff, Halogen, Nitro, Cyan, $C_1$—$C_4$-Alkyl, $C_1$—$C_4$-Alkoxy oder $C_1$—$C_3$-Acyl,

$R_4$, $R_5$ und $R_6$ unabhängig voneinander Wasserstoff, Halogen oder $C_1$—$C_4$-Alkyl,

X und Y zusammen einen aliphatischen, gesättigten oder ungesättigten geradkettigen oder verzweigten Kohlenwasserstoffrest mit bis zu 12 Kohlenstoffatomen, mit der Massgabe, dass im Falle der ungesättigten Reste die Mehrfachbindung durch mindestens ein an der Mehrfachbindung nicht beteiligtes Kohlenstoffatom vom Sauerstoffatom getrennt ist, oder

X Methylen und

Y einen Dioxolanrest, einen Dioxanrest oder einen Tetrahydrofuranrest, die gegebenenfalls durch Alkyl mit 1 bis 4 C-Atomen substituiert sind, bedeutet, unter Einschluss ihrer Säureadditionssalze und Metallkomplexe, oder einem Mittel, welches eine dieser Verbindungen enthält, behandelt.

2. Verfahren nach Anspruch 1, dadurch gekennzeichnet, dass man eine Verbindung der Formel I, in welcher $R_1$, $R_2$, $R_4$ und $R_5$ Wasserstoff, $R_3$ Wasserstoff oder Chlor, $R_6$ Wasserstoff oder Methyl und i) X Methylen und Y 1,3-Dioxolan-2-yl oder ii) Y Wasserstoff und X und Y gemeinsam n-Octyl oder 2-Butenyl bedeuten, verwendet.

3. Verfahren nach Anspruch 1 zum Schützen von Getreide.

4. Verbindungen der Formel I gemäss Anspruch 1

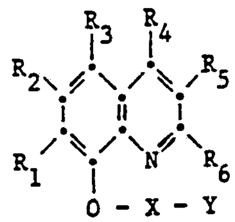

(I),

worin die Reste $R_1$ bis $R_6$, X und Y wie in Anspruch 1 definiert sind unter Einschluss ihrer Saäureadditionssalze und Metallkomplexe, mit der Massgabe, dass nachstehend genannte Verbindungen nicht umfasst sind:

8-(3,7-Dimethyl-oct-6-enyl-pxy)-chinolin,
8-(3,7-Dimethyl-octa-2,6-dienyl-oxy)-chinolin,
8-Allyloxychinolin, 8-Allyloxy-4-methylchinolin und 8-Allyloxy-2-methylchinolin.

5. Verbindungen der Formel Ia gemäss Anspruch 4

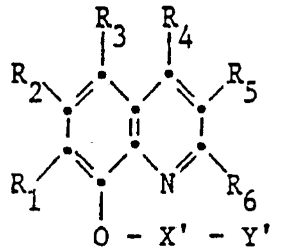

(Ia),

worin die Substituentengruppe —X'—Y' 2-Butenyl, 1,3-Dioxolan-2-ylmethyl, 1,3-Dioxan-2-ylmethyl oder Tetrahydrofuran-2-ylmethyl bedeuten.

6. 5-Chlor-6-allyloxychinolin, 5-Chlor-8-propargyloxychinolin oder 5-Chlor-8-(2-butenyloxy)-chinolin als Verbindungen der Formel I.

7. Mittel zum Schützen von Kulturpflanzen gegen schädigende Wirkungen von Herbiziden, dadurch gekennzeichnet, dass es als aktive Komponente eine Verbindung der Formel I nach Anspruch 1 enthält.

8. Mittel nach Anspruch 7, dadurch gekennzeichnet, dass es eine Verbindung der Formel I zusammen mit inertem Zusatzmaterial enthält.

9. Mittel nach Anspruch 7, dadurch gekennzeichnet, dass es eine Verbindung der Formel I zusammen mit dem zu antagonisierenden Herbizid enthält.

10. Mittel nach Anspruch 7, dadurch gekennzeichnet, dass es 0,1 bis 99 Gewichtsprozent Wirkstoff der Formel I oder eine Kombination von Wirkstoff der Formel I und zu antagonisierendem Herbizid und 1 bis 99,9 Gewichtsprozent eines festen oder flüssigen Zusatzstoffes, davon 0 bis 25 Gewichtsprozent eines Tensides, enthält.

11. Mittel nach Anspruch 10, dadurch gekennzeichnet, dass es 0,1 bis 95 Gewichtsprozent Wirkstoff der Formel I oder eine Kombination von Wirkstoff der Formel I und zu antagonisierendem Herbizid, 5 bis 99,8 Gewichtsprozent eines festen oder flüssigen Zusatzstoffes und 0,1 bis 25 Gewichtsprozent eines Tensides enthält.

12. Verfahren zur Herstellung neuer Chinolinderivate der Formel I gemäss Anspruch 4, dadurch gekennzeichnet, dass man eine Verbindung der Formel II

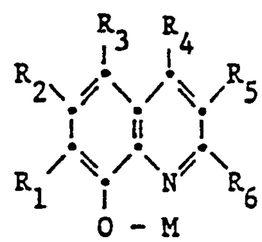

(II),

worin $R_1$, $R_2$, $R_3$, $R_4$, $R_5$ und $R_6$ die für Formel Ia angegebenen Bedeutungen haben und M für Wasserstoff, ein Alkali- oder Erdalkalimetallatom steht, mit einer Verbindung der Formel III

$$Z—X—Y \quad \text{(III)},$$

worin X und Y die für Formel I angegebenen Bedeutungen haben und Z für einen abspaltbaren Rest steht, umsetzt.

**Revendications**

1. Procédé de protection des plantes cultivées contre les effets nocifs des herbicides, caractérisé en ce qu'on traite les plantes cultivées, des parties de ces plantes ou des surfaces prévues pour ces plantes cultivées avec un composé de formule I

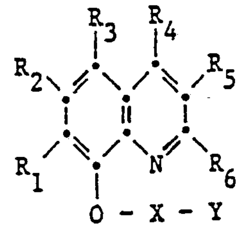

(I),

où

$R_1$ $R_2$ et $R_3$ représentent indépendamment l'un de l'autre un hydrogène, halogène, nitro, cyano, alcoyle en $C_{1-4}$, alcoxy en $C_{1-4}$ ou acyle en $C_{1-3}$,

$R_4$, $R_5$ et $R_6$ représentent indépendamment l'un de l'autre un hydrogène, halogène, ou alcoyle en $C_{1-4}$,

X et Y représentent ensemble un radical hydrocarboné aliphatique, saturé ou non saturé, à chaîne droite ou ramifiée, ayant jusqu'à 12 atomes de carbone, avec cette précision que dans le cas des radicaux non saturés la liaison multiple est séparée de l'atome d'oxygène par au moins un atome de carbone ne participant pas à la liaison multiple, ou

X représente un méthylène et

Y représente un radical dioxolane, un radical dioxanne ou un radical tétrahydrofuranne, qui sont éventuellement substitués par un alcoyle en $C_{1-4}$, y compris ses sels d'addition d'acides et complexes métalliques ou un agent qui contient un de ces composés.

2. Procédé selon la revendication 1, caractérisé en ce qu'on utilise un composé de formule I où $R_1$, $R_2$, $R_4$ et $R_5$ représentent un hydrogène, $R_3$ un hydrogène ou un chlore, $R_6$ un hydrogène ou un méthyle et

i) X est un méthylène et Y un 1,3-dioxolan-2-yle ou

ii) Y un hydrogène et X et Y ensemble un n-octyle ou un 2-butényle.

3. Procédé selon la revendication 1, de protection des céréales.

4. Composés de formule I selon la revendication 1

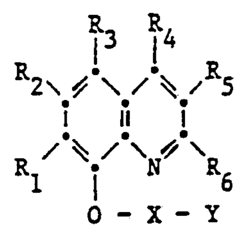

(I),

où

les radicaux $R_1$ à $R_6$, X et Y sont tels que définis dans la revendication 1 y compris leurs sels d'addition d'acides et complexes métalliques, avec cette précision que les composés mentionnés ci-dessous ne sont pas compris:

8-(3,7-diméthyl-oct-6-ényl-oxy)-quinoléine,
8-(3,7-diméthyl-octa-2,6-diényl-oxy)-quinoléine,
8-allyloxyquinoléine, 8-allyloxy-4-méthylquinoléine
et 8-allyloxy-2-méthylquinoléine.

5. Composés de formule Ia selon la revendication 4

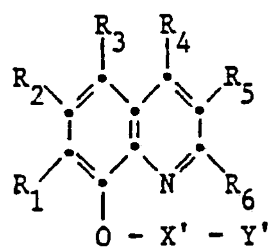

(Ia),

où

le groupe de substituants —X'—Y' représente un 2-butényle, 1,3-dioxolane-2-ylméthyle, 1,3-dioxan-2-ylméthyle ou tétrahydrofuran-2-ylméthyle.

6. 5-chloro-6-allyloxyquinoléine, 5-chloro-8-propargyloxyquinoléine ou 5-chloro-8-(2-buténytoxy)quinoléine comme composés de formule I.

7. Agent de protection des plantes cultivées contre les effets nocifs des herbicides, caractérisé en ce qu'il contient comme composant actif un composé de formule I selon la revendication 1.

8. Agent selon la revendication 7, caractérisé en ce qu'il contient un composé de formule I avec un additif inerte.

9. Agent selon la revendication 7, caractérisé en ce qu'il contient un composé de formule I avec l'herbicide auquel il s'agit de s'opposer.

10. Agent selon la revendication 7, caractérisé en ce qu'il contient de 0,1 à 99% de matière active de formule I ou une combinaison de matière active de formule I et d'herbicide auquel il s'agit de s'opposer et de 1 à 99,9% en poids d'un additif solide ou liquide, dont 0 à 25% en poids d'un agent tensio-actif.

11. Agent selon la revendication 10, caractérisé en ce qu'il contient de 0,1 à 95% en poids de matière active de formule I ou d'une combinaison de matière active de formule I et d'un herbicide auquel il s'agit de s'opposer, de 5 à 99,8% en poids d'un additif solide ou liquide et de 0,1 à 25% en poids d'un agent tensio-actif.

12. Procédé de préparation de nouveaux dérivés de quinoléine de formule I selon la revendication 4, caractérisé en ce qu'on fait réagir un composé de formule II

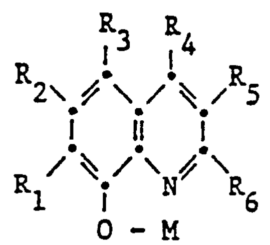

(II),

où $R_1$, $R_2$, $R_3$, $R_4$, $R_5$ et $R_6$ ont les significations données pour la formule Ia et M représente un hydrogène, un atome de métal alcalin ou alcalino-terreux, avec un composé de formule III

$$Z—X—Y \quad (III)$$

où X et Y ont les significations données pour la formule I et Z représente un radical séparable.

**Claims**

1. A method for the protection of cultivated plants against harmful effects of herbicides, which method comprises treating the cultivated plants, parts of these plants, or cultivated area for cultivated plants, with a compound of the formula I

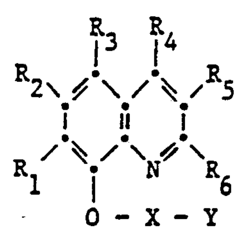

(I),

in which

$R_1$, $R_2$ and $R_3$ independently of one another are each hydrogen, halogen, nitro, cyano, $C_1$—$C_4$alkyl, $C_1$—$C_4$alkoxy or $C_1$—$C_3$acyl,

$R_4$, $R_5$ and $R_6$ independently of one another are each hydrogen, halogen or $C_1$—$C_4$alkyl,

X and Y together are an aliphatic, saturated or unsaturated, straight-chain or branched hydrocarbon radical which has up to 12 carbon atoms, with the proviso that in the case of the unsaturated radicals the multiple bond is separated from the oxygen atom by at least one carbon atom not participating in the multiple bond, or

X is methylene and

Y is a dioxolane radical, a dioxane radical or a tetrahydrofuran radical, which are unsubstituted or substituted by alkyl having 1 to 4 carbon atoms, with the inclusion of the acid addition salts and metal complexes thereof, or with a composition containing one of these compounds.

2. A method according to claim 1, wherein there is used a compound of the formula I in which $R_1$, $R_2$, $R_4$ and $R_5$ are hydrogen, $R_3$ is hydrogen or chlorine, $R_6$ is hydrogen or methyl, and i) X is methylene and Y is 1,3-dioxolan-2-yl or ii) Y is hydrogen and X and Y tegether are n-octyl or 2-butenyl.

3. A method according to claim 1 for the protection of cereal crops.

4. A compound of the formula I according to claim 1

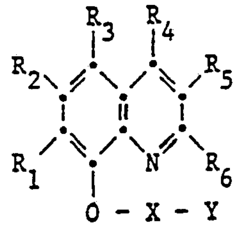

(I),

in which
the radicals $R_1$ to $R_6$, X and Y are as defined in claim 1, with the inclusion of the acid addition salts and metal complexes therof, with the proviso that the compounds mentioned below are not included:
8-(3,7-dimethyloct-6-enyloxy)-quinoline,
8-(3,7-dimethylocta-2,6-dienyloxy)-quinoline,
6-allyloxyquinoline, 8-allyloxy-4-methylquinoline and 8-allyloxy-2-methylquinoline.

5. A compound of the formula Ia according to claim 4

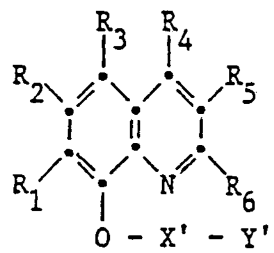

(Ia),

in which
the substituent group —X'—Y' is 2-butenyl, 1,3-dioxolan-2-ylmethyl, 1,3-dioxan-2-ylmethyl or tetrahydrofuran-2-ylmethyl.

6. 5-chloro-6-allyloxyquinoline, 5-chloro-8-propargyloxyquinoline or 5-chloro-8-(2-butenyloxy)-quinoline as compounds of the formula I.

7. A composition for the protection of cultivated plants against the harmful effects of herbicides, which composition contains as active component a compound of the formula I according to claim 1.

8. A composition according to claim 7, which contains a compound of the formula I together with inert additive material.

9. A composition according to claim 7, which contains a compound of the formula I together with the herbicide to be antagonised.

10. A composition according to claim 7, which contains 0.1 to 99 per cent by weight of active ingredient of the formula I, or a combination of active ingredient of the formula I and herbicide to be antagonised, and 1 to 99.9 per cent by weight of a solid or liquid additive, of which 0 to 25 per cent by weight is a surfactant.

11. A composition according to claim 10, which contains 0.1 to 95 per cent by weight of active ingredient of the formula I, or a combination of active ingredient of the formula I and herbicide to be antagonised, 5 to 99.8 per cent by weight of a solid or liquid additive, and 0.1 to 25 per cent by weight of a surfactant.

12. A process for the preparation of a novel quinoline derivative of the formula I according to claim 4, which process comprises reacting a compound of the formula II

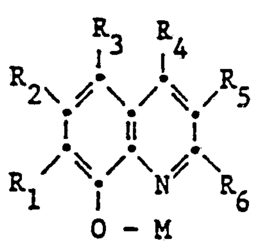

(II),

in which $R_1$, $R_2$, $R_3$, $R_4$, $R_5$ and $R_6$ have the meanings defined under formula Ia, and M is hydrogen, an alkali metal or alkaline earth metal atom, with a compound of the formula III

Z—X—Y (III),

in which X and Y have the meanings defined for formula I, and Z is a detachable radical.